# FASCICULE DE BREVET EUROPEEN

(11) **EP 1 739 074 B1**
(45) Date de publication et mention de la délivrance du brevet: **12.03.2008**
(21) Numéro de dépôt: 06116072.7
(22) Date de dépôt: 26.06.2006
(51) Int. Cl.: C07C 211/53, A61K 8/41

(54) **Nouvelles para-phénylènediamines doubles reliées par un groupe aliphatique ramifié et utilisation en coloration**
Neue Bis-phenylendiamine verbunden mit einer verzweigten aliphatischen Gruppe, sowie ihre Verwendung als Färbemittel
Novel double para-phenylenediamines joined by a branched aliphatic group and use in dyeing

(30) Priorité: 29.06.2005 FR 0551807
(43) Date de publication de la demande: 03.01.2007
(73) Titulaire: L'ORÉAL, 75008 Paris (FR)
(72) Inventeur: Sabelle, Stéphane, 75005, PARIS (FR); Metais, Eric, 95320, St Leu la Forêt (FR)
(74) Mandataire: Fevrier, Murielle Françoise E.

(56) Documents cités:
- WO-A-01/72686
- WO-A-02/06207
- FR-A- 2 016 123
- DATABASE CA [Online] CHEMICAL ABSTRACTS SERVICE, COLUMBUS, OHIO, US; HAYASHIDA, SHIGERU ET AL: "heat-resistant active waveguide composition, its active waveguide, and its manufacture" XP002379768 extrait de STN Database accession no. 1996:379311 & JP 08 041329 A2 (HITACHI CHEMICAL CO LTD, JAPAN) 13 février 1996 (1996-02-13)

## Description

La présente demande concerne une nouvelle famille de para-phénylènediamines doubles reliées par un bras de liaison aliphatique ramifié et leur utilisation pour la coloration des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques et pyridiniques.

La variété des molécules mises en jeu au niveau des bases d'oxydation et des coupleurs, permet l'obtention d'une riche palette de couleurs.

Le brevet FR-A-2 016 123 (L'OREAL) divulgue des bases oxydantes du type bis-paraphénylènediamines présentant un "linker" alkyle non ramifié.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

Le but de la présente invention est de fournir de nouvelles bases d'oxydation capables de colorer les fibres kératiniques avec des nuances variées, puissantes, esthétiques et peu sélectives ainsi que des colorations résistantes aux diverses agressions que peuvent subir les fibres telles que la lumière, la sueur et les shampoings.

Ce but est atteint avec la présente invention qui a pour objet une nouvelle famille de paraphénylènediamine double de formule (I) suivante ainsi que les sels d'addition correspondants: 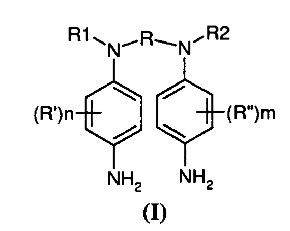 dans laquelle :
- R représente un radical alkylène en C₃-C₁₅ ramifié,
- R₁ et R₂ représentent, indépendamment l'un de l'autre,
   - un atome d'hydrogène
   - un radical alkyle en C₁-C₆ linéaire ou ramifié,
   - un radical alkyle en C₁-C₆ linéaire ou ramifié substitué par un ou plusieurs radicaux hydroxy, alkoxy en C₁-C₄, amino, monoalkyl amino en C₁-C₄, dialkylamino en C₁-C₄,
- R' et R" représentent, indépendamment l'un de l'autre,
   - un radical alkyle en C₁-C₆,
   - un radical alcoxy en C₁-C₆,
   - un radical hydroxy-alcoxy en C₁-C₆,
   - un radical alcoxy(C₁-C₆)alkyle en C₁-C₆,
   - un radical mono ou poly-hydroxy alkyle en C₁-C₆,
- n et m représentent, indépendamment l'un de l'autre, un entier compris entre 0 et 4.

Les para-phénylènediamines de formule (I) permettent en particulier d'obtenir une coloration de fibres kératiniques très puissante, peu sélective et tenace, en particulier à la lumière.

Un autre objet de l'invention concerne des compositions pour la coloration des fibres kératiniques, en particulier des fibres kératiniques humaines telles que les cheveux, comprenant au moins une para-phénylènediamine de formule (I).

L'invention a aussi pour objet un procédé de coloration mettant en oeuvre cette composition, l'utilisation de la composition selon la présente invention pour la coloration des fibres kératiniques, en particulier les fibres kératiniques humaines telles que les cheveux et un dispositif à plusieurs compartiments ou "kit" de teinture.

A titre d'exemple de para-phénylènediamines de formule (I), on peut citer les para-phénylènediamines suivantes :

| | |
|---|---|
| 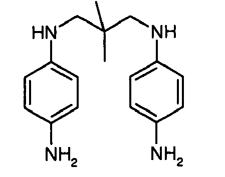 | 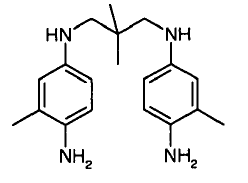 |
| 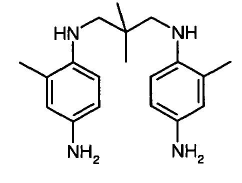 | 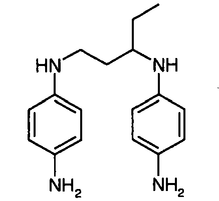 |
| 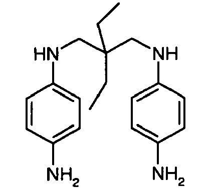 | 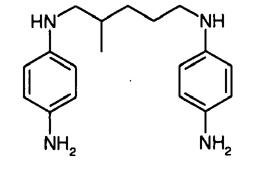 |
| 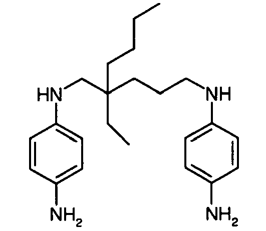 | 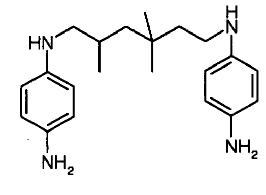 |
| 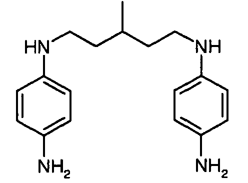 | |

Selon un mode de réalisation particulier, les para-phénylènediamines de formule (I) sont telles que R est un radical alkylène ramifié en C₄-C₁₁. R₁ et R₂ représentent indépendamment un atome d'hydrogène ou un groupe alkyle en C₁-C₄ pouvant être substitué. n et m sont de préférence égal à 0 ou 1.

D'une manière générale, les sels d'addition utilisables sont notamment choisis parmi les sels d'addition avec un acide, tels que l'acide chlorhydrique, l'acide bromhydrique, l'acide sulfurique, l'acide citrique,l'acide succinique, l'acide tartrique, l'acide lactique, l'acide paratoluènesulfonique, l'acide benzènesulfonique, l'acide phosphorique et l'acide acétique.

Ils peuvent aussi être sous forme de solvates par exemple un hydrate ou un solvate d'alcool linéaire ou ramifié tel que l'éthanol ou l'isopropanol.

Les para-phénylènediamines de formule (I) selon la présente demande peuvent être préparés suivant une méthode de synthèse classique. On pourra par exemple se reporter à la demande de brevet DE10144226A.

A titre d'illustration, les para-phénylènediamines de formule (I) peuvent être synthétisés selon le schéma réactionnel suivant :

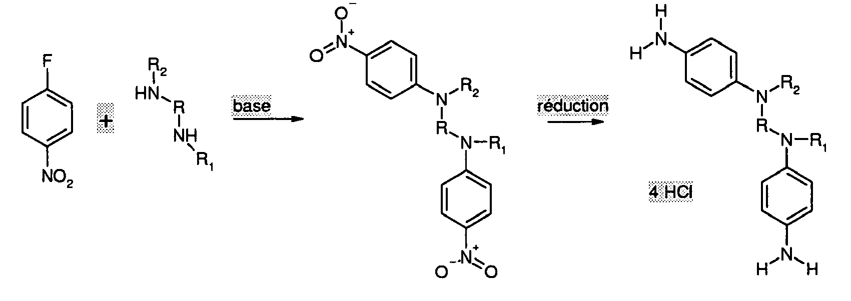

La première étape de la synthèse est une substitution nucléophile d'une diamine sur un dérivé de para-fluoro-nitrobenzène, étape inspirée des publications Synthesis 1990 (12), 1147-1148 et Synth. Commun. 1990, 20 (22), 3537-3545. La deuxième étape est une étape de réduction classique, pouvant être par exemple une réaction d'hydrogénation par catalyse hétérogène en présence de Pd/C, Pd(II)/C, Ni de Raney ou encore une réaction de réduction par un métal, par exemple par du zinc, fer, étain... (Advanced Organic Chemistry, 4th edition, 1992, J. MARCH, WILEY Interscience ; Reduction in Organic Chemistry, M. Hudlicky, 1983, Ellis Honwood series Chemical Science).

La présente demande concerne aussi les composés nitrés de formule (II) suivante qui peuvent être utilisés pour l'obtention des para-phénylènediamines de formule (I) : 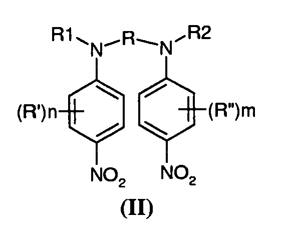 dans laquelle R₁, R₂, R, R', R", n, m sont tels que définis précédemment à l'exception du 1,2-propanediamine N, N'-bis(4-nitrophenyl).

L'invention a aussi pour objet une composition de teinture comprenant dans un milieu approprié à la teinture au moins une base d'oxydation du type para-phénylènediamine de formule (I) telle que définie précédemment.

La quantité en para-phénylènediamine de formule (I) dans la composition de teinture est en général comprise entre 0,0001% et 20% en poids par rapport au poids total de la composition, de préférence entre 0,01% et 10%.

La composition selon l'invention contient de préférence au moins un coupleur d'oxydation.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement, la quantité du ou des coupleurs d'oxydation est comprise entre 0,0001 et 20%, de préférence entre 0,005 à 6% en poids par rapport au poids total de la composition.

La composition selon l'invention peut également contenir au moins une base d'oxydation additionnelle différente des bases d'oxydation de formule (I).

Les bases d'oxydation peuvent notamment être choisies parmi les para-phénylènediamines, les bis-phénylalkylènediamines, les para--aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro paraphénylènediamine, la 2,3-diméthyl para-phénylènediamine, la 2,6-diméthyl paraphénylènediamine, la 2,6-diéthyl para-phénylènediamine, la 2,5-diméthyl paraphénylènediamine, la N,N-diméthyl para-phénylènediamine, la N,N-diéthyl paraphénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy paraphénylènediamine, la N-(β-méthoxyéthyl) para-phénylènediamine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl paraphénylènediamine, la 2-β-hydroxyéthyloxy para-phénylènediamine, la 2,6-diméthyl paraphénylènediamine, la 2,6-diéthyl para-phénylènediamine, la 2,3-diméthyl paraphénylènediamine, la N,N-bis-(β-hydroxyéthyl) para-phénylènediamine, la 2-chloro paraphénylènediamine, la 2-β-acétylaminoéthyloxy para-phénylènediamine, la 6-(4-Aminophenylamino)-hexan-1-ol et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4'-aminophényl) tétraméthylènediamine, la N,N'-bis-(β-hydroxyéthyl) N,N'-bis-(4-aminophényl) tétraméthylènediamine, la N,N'-bis-(4-amino 3'-methylphényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, le 5-[(2-hydroxyethyl)amino]-2-methylphenol et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2733749 et DE 19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

La composition tinctoriale conforme à l'invention peut en outre contenir un ou plusieurs colorants directs pouvant notamment être choisi parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40% en poids environ par rapport au poids total de la composition tinctoriale, et encore plus préférentiellement entre 5 et 30% en poids environ.

Avantageusement, la composition de teinture comprend au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20% en poids par rapport au poids de la composition.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 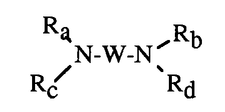 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de la présente demande concerne un procédé de teinture des fibres kératiniques dans lequel on applique sur les fibres la composition de l'invention telle que définie précédemment, et on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement à la composition de l'invention.

A titre d'agents oxydants, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, notamment humaines et en particulier les cheveux humains.

La présente demande concerne également l'utilisation de la composition cosmétique selon l'invention comprenant, dans un milieu approprié pour la teinture, au moins une paraphénylènediamine de formule (I) pour la teinture des fibres kératiniques, de préférence les fibres kératiniques humaines telles que les cheveux.

L'invention a aussi pour objet un dispositif à plusieurs compartiments ou "kit" de teinture dans lequel un premier compartiment renferme une composition tinctoriale contenant au moins une para-phénylènediamine de formule (I) définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2586913.

### EXEMPLES DE SYNTHESE

### EXEMPLE 1 : Synthèse du tétrachlorhydrate de la 2-2-diméthyl-N,N'-bis(4-aminophényl)propane-1,3-diamine (2)

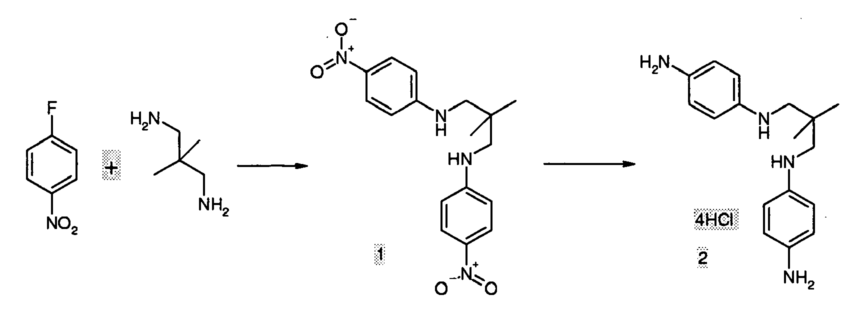

### Synthèse de la 2,2-diméthyl-N,N'-bis(4-nitrophényl)propane-1,3-diamine (1)

2,3 g de 4-fluoronitrobenzène (2 éq.) sont dissous dans 10 ml de DMSO. 1,2 équivalent de 2,2-diméthyl-1,3-propanediamine et 4 équivalents de triéthylamine sont ajoutés à la solution. Le milieu réactionnel est porté à 60°C pendant 20 heures. Le mélange est ensuite versé sur de la glace pilée, un précipité se forme. Ce dernier est filtré, lavé à l'eau, puis séché.

### Synthèse du tétrachlorhydrate de la 2,2-diméthyl-N,N'-bis(4-aminophényl)propane-1,3-diamine (2)

La 2,2-dimethyl-N,N'-bis(4-nitrophényl)propane-1,3-diamine (1) obtenue lors de l'étape précédente est réduite avec un mélange zinc/chlorure d'ammonium/eau/éthanol bouillant. L'amine correspondante est isolée sous forme de tétrachlorhydrate.

Les spectres RMN du proton et de masse sont conformes à la structure attendue du produit.

### EXEMPLE 2 : Synthèse du tétrachlorhydrate de la 2,2-diméthyl-N,N'-bis(3-méthyl-4-aminophényl)propane-1,3-diamine (4)

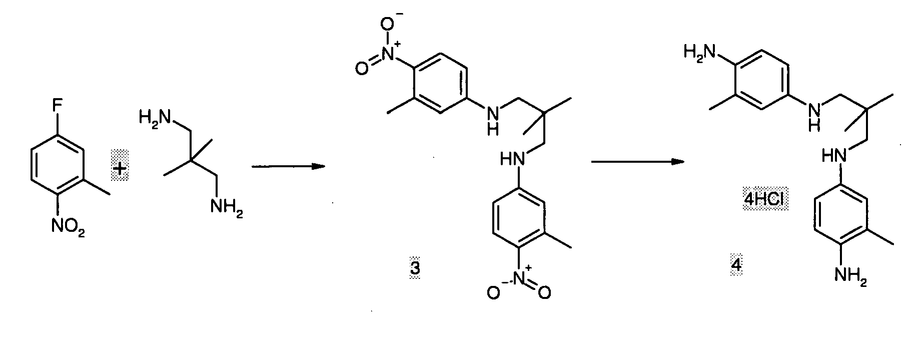

### Synthèse du 2,2-diméthyl-N,N'-bis(3-méthyl-4-nitrophényl)propane-1,3-diamine (3)

1,5 g de 5-fluoro-2-nitrotoluène (2 éq.) sont dissous dans 10 ml de DMSO. 1,2 équivalent de 2,2-diméthyl-1,3-propanediamine et 1,2 équivalents de triéthylamine sont ajoutés à la solution. Le milieu réactionnel est porté à 80°C pendant 24 heures. Le mélange est ensuite versé sur de la glace pilée, un précipité se forme. Ce dernier est filtré, lavé à l'eau, puis séché.

### Synthèse du tétrachlorhydrate de la 2,2-diméthyl-N,N'-bis(3-méthyl-4-aminophényl)propane-1,3-diamine (4)

La 2,2-diméthyl-N,N'-bis(3-méthyl-4-nitrophényl) propane-1,3-diamine (3) obtenue lors de l'étape précédente est réduite avec un mélange zinc/chlorure d'ammonium/eau/éthanol bouillant. L'amine correspondante est isolée sous forme de tétrachlorhydrate.

Les spectres RMN du proton et de masse sont conformes à la structure attendue du produit.

### EXEMPLE 3 : Synthèse du tétrachlorhydrate de la 2,2-dimethyl-N,N'-bis(2-méthyl-4-aminophényl)propane-1,3-diamine (6)

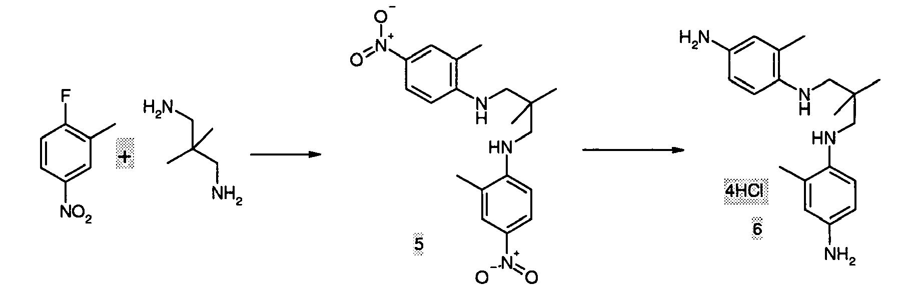

### Synthèse de la 2,2-diméthyl-N,N'-bis(2-méthyl-4-nitrophényl)propane-1,3-diamine (5)

1,5 g de 2-fluoro-5-nitrotoluène (2 éq.) sont dissous dans 10 ml de DMSO. 1,2 équivalent de 2,2-diméthyl-1,3-propanediamine et 1,2 équivalents de triéthylamine sont ajoutés à la solution. Le milieu réactionnel est porté à 80°C pendant 24 heures. Le mélange est ensuite versé sur de la glace pilée, un précipité se forme. Ce dernier est filtré, lavé à l'eau, puis séché.

### Synthèse du tétrachlorhydrate de la 2,2-diméthyl-N,N'-bis(2-méthyl-4-aminophényl)propane-1,3-diamine (6)

La 2,2-diméthyl-N,N'-bis(2-méthyl-4-nitrophényl)propane-1,3-diamine (5) obtenue lors de l'étape précédente est réduite avec un mélange zinc/chlorure d'ammonium/eau/éthanol bouillant. L'amine correspondante est isolée sous forme de tétrachlorhydrate.

Les spectres RMN du proton et de masse sont conformes à la structure attendue du produit.

### EXEMPLE 4: Synthèse du tétrachlorhydrate de la N-{-3[(4-aminophényl)amino]-1-éthylpropyl}benzène-1,4-diamine (8)

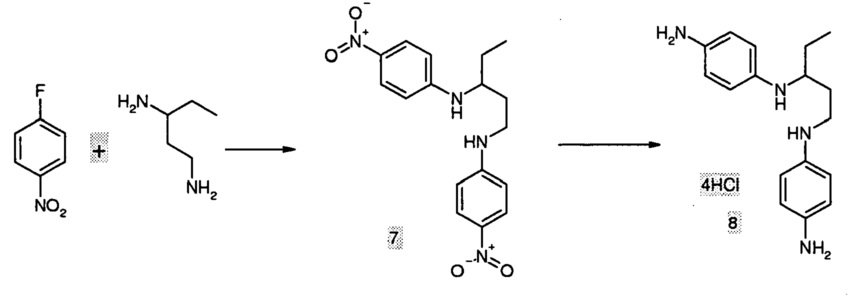

### Synthèse de la N,N'-bis(4-nitrophényl)pentane-1,3-diamine (7)

2,3 g de 4-fluoronitrobenzène (2 éq.) sont dissous dans 10 ml de DMSO. 1,2 équivalents de 3-amino-1-éthylpropylamine et 4 équivalents de triéthylamine sont ajoutés à la solution. Le milieu réactionnel est porté à 60°C pendant 16 heures. Le mélange est ensuite versé sur de la glace pilée, un précipité se forme. Ce dernier est filtré, lavé à l'eau, puis séché.

### Synthèse du tétrachlorhydrate de la N-{3-[(4-aminophényl)amino]-1-éthylpropyl}benzène-1,4-diamine (8)

La N,N'-bis(4-nitrophényl)pentane-1,3-diamine (7) obtenue lors de l'étape précédente est réduite avec un mélange zinc / chlorure d'ammonium / eau/ éthanol bouillant. L'amine correspondante est isolée sous forme de tétrachlorhydrate.

Les spectres RMN du proton et de masse sont conformes à la structure attendue du produit.

### EXEMPLE 5 : Synthèse du tétrachlorhydrate de la N-(4-aminophényl-N-{5-[(4-aminophényl)amino]-2-méthylpentyl}amine (10)

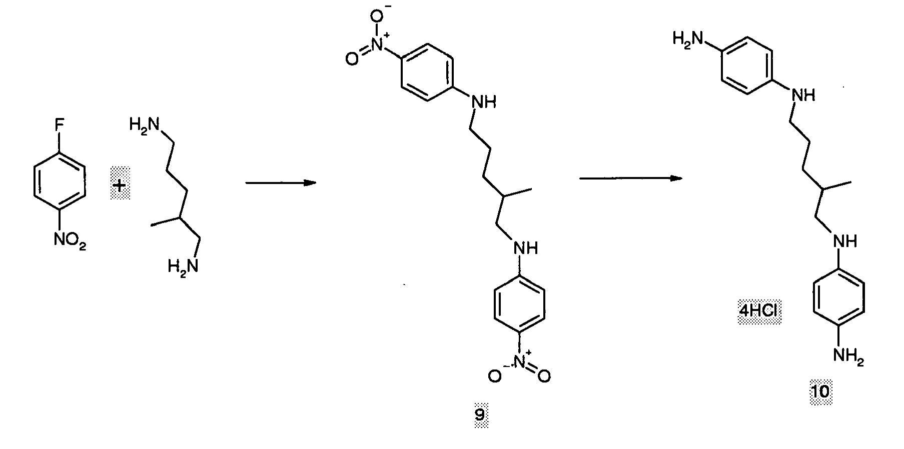

### Synthèse de la N-{2-methyl-5-[(4-nitrophényl)amino]pentyl}-N-(4-nitrophényl)amine (9)

2,3 g de 4-fluoronitrobenzène (2 éq.) sont dissous dans 10 ml de DMSO. 1,2 équivalent de 2-méthylpentane-1,5-diamine et 4 équivalents de triéthylamine sont ajoutés à la solution. Le milieu réactionnel est porté à 60°C pendant 20 heures. Le mélange est ensuite versé sur de la glace pilée, un précipité se forme. Ce dernier est filtré, lavé à l'eau, puis séché.

### Synthèse du tétrachlorhydrate de la N-(4-aminophényl)-N-{5-[(4-aminophényl)amino]-2-méthylpentyl}amine (10)

La N-{2-méthyl-5-[(4-nitrophényl)amino]pentyl}-N-(4-nitrophényl) amine obtenue lors de l'étape précédente est réduite avec un mélange zinc / chlorure d'ammonium / eau/ éthanol bouillant. L'amine correspondante est isolée sous forme de tétrachlorhydrate.

Les spectres RMN du proton et de masse sont conformes à la structure attendue du produit.

### EXEMPLES DE COMPOSITIONS TINCTORIALES

### Exemples 1 à 14 : Composition tinctoriale à partir du tétrachlorhydrate de la 2,2-diméthyl-N,N'-bis(4-aminophényl)propane-1,3-diamine (2)

### Exemples 1 à 7 : Teinture en milieu acide

Les compositions tinctoriales suivantes sont préparées :

| Exemple | 1 | 2 | 3 | 4 | 5 | 6 | 7 |
|---|---|---|---|---|---|---|---|
| tétrachlorhydrate de la 2,2-diméthyl-N,N'-bis(4- aminophényl)propane-1,3- diamine (2) | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H- pyrazolo[5,1-c][1,2 ,4]triazole | | | | | 10⁻³ mole | | |
| chlorhydrate du 2-(2,4- Diamino-phénoxy)-éthanol | | | | | | 10⁻³ mole | |
| chlorhydrate du 3-Amino- 2-chloro-6-méthyl-phénol | | | | | | | 10⁻³ mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.
Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 1 | 2 | 3 | 4 | 5 | 6 | 7 |
|---|---|---|---|---|---|---|---|
| Nuance observée | brun | gris violet- rouge intense | brun intense | brun rouge intense | brun rouge | bleu intense | violet intense |

### Exemples 8 à 14 : teinture en milieu basique

Les compositions tinctoriales suivantes sont préparées :

| Exemple | 8 | 9 | 10 | 11 | 12 | 13 | 14 |
|---|---|---|---|---|---|---|---|
| tétrachlorhydrate de la 2,2-diméthyl-N,N'-bis(4- aminophényl)propane-1,3- diamine (2) | 10⁻³ mole | 10 ⁻³ mole | 10⁻³ mole | 10⁻³ mole | 10⁻³ mole | 10 ⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H-pyrazolo[5,1- c][1,2,4]triazole | | | | | 10⁻³ mole | | |
| chlorhydrate du 2-(2,4- Diamino-phénoxy)- éthanol | | | | | | 10⁻³ mole | |
| chlorhydrate du 3-Amino- 2-chloro-6-méthyl-phénol | | | | | | | 10⁻³ mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (2) pH 9.5 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 8 | 9 | 10 | 11 | 12 | 13 | 14 |
|---|---|---|---|---|---|---|---|
| Nuance observée | brun | gris violet intense | rouge intense | brun rouge intense | violet- rouge intense | bleu intense | violet intense |

### Exemples 15 à 25 : Composition tinctoriale à partir du tétrachlorhydrate de la 2,2-diméthyl-N,N'-bis(3-méthyl-4-aminophényl)propane-1,3-diamine (4)

### Exemples 15 à 19 : Teinture en milieu acide

Les compositions tinctoriales suivantes sont préparées :

| Exemple | 15 | 16 | 17 | 18 | 19 |
|---|---|---|---|---|---|
| tétrachlorhydrate de la 2,2-diméthyl- N,N'-bis(3-méthyl-4- aminophényl)propane-1,3-diamine (4) | 10⁻³ mole | 10⁻³ mole | 10⁻³ mole | 10⁻³ mole | 10⁻³ mole |
| 5-Amino-2-méthyl-phénol | 10⁻³ mole | | | | |
| 1H-Indol-6-ol | | 10⁻³ mole | | | |
| 2-Amino-pyridin-3-ol | | | 10⁻³ mole | | |
| chlorhydrate du 2-(2,4-Diamino- phénoxy)-éthanol | | | | 10⁻³ mole | |
| chlorhydrate du 3-Amino-2-chloro-6-méthyl-phénol | | | | | 10⁻³ mole |
| Support de teinture (1) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) tel que défini précédemment | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 15 | 16 | 17 | 18 | 19 |
|---|---|---|---|---|---|
| Nuance observée | gris violet | brun | jaune | bleu intense | violet-bleu intense |

### Exemples 20 à 25 : teinture en milieu basique

Les compositions tinctoriales suivantes sont préparées :

| Exemple | 20 | 21 | 22 | 23 | 24 | 25 |
|---|---|---|---|---|---|---|
| tétrachlorhydrate de la 2,2-diméthyl-N,N'-bis(3-méthyl-4- aminophényl)propane-1,3-diamine(4) | 10⁻³ mole | 10⁻³ mole | 10⁻³ mole | 10⁻³ mole | 10⁻³ mole | 10⁻³ mole |
| 5-Amino-2-méthyl-phenol | 10⁻³ mole | | | | | |
| 1H-Indol-6-ol | | 10⁻³ mole | | | | |
| 2-Amino-pyridin-3-ol | | | 10⁻³ mole | | | |
| 3,6-Diméthyl-1H-pyrazolo[5,1-c][1,2 ,4] triazole | | | | 10⁻³ mole | | |
| chlorhydrate du 2-(2,4-Diamino-phénoxy)- éthanol | | | | | 10⁻³ mole | |
| chlorhydrate du 3-Amino- 2-chloro-6-methyl-phénol | | | | | | 10⁻³ mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) tel que défini précédemment | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 20 | 21 | 22 | 23 | 24 | 25 |
|---|---|---|---|---|---|---|
| Nuance observée | violet intense | rouge | orangé | rouge | bleu intense | gris violet-bleu |

### Exemples 26 à 36 : Composition tinctoriale à partir du tétrachlorhydrate de la 2,2-diméthyl-N,N'-bis(2-méthyl-4-aminophényl)propane-1,3-diamine (6)

### Exemples 26 à 30 : Teinture en milieu acide

Les compositions tinctoriales suivantes sont préparées :

| Exemple | 26 | 27 | 28 | 29 | 30 |
|---|---|---|---|---|---|
| tétrachlorhydrate de la 2,2-diméthyl-N,N'-bis(2-méthyl-4- aminophényl)propane-1,3-diamine (6) | 10⁻³ mole | 10⁻³ mole | 10⁻³ mole | 10⁻³ mole | 10⁻³ mole |
| 5-Amino-2-méthyl-phénol | 10⁻³ mole | | | | |
| 1H-Indol-6-ol | | 10⁻³ mole | | | |
| 2-Amino-pyridin-3-ol | | | 10⁻³ mole | | |
| chlorhydrate du 2-(2,4-Diamino- phénoxy)-éthanol | | | | 10⁻³ mole | |
| chlorhydrate du 3-Amino-2- chloro-6-méthyl-phénol | | | | | 10⁻³ mole |
| Support de teinture (1) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) tel que défini précédemment | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 26 | 27 | 28 | 29 | 30 |
|---|---|---|---|---|---|
| Nuance observée | gris violet- rouge | gris intense | brun orangé | bleu intense | violet intense |

### Exemples 31 à 35 : teinture en milieu basique

Les compositions tinctoriales suivantes sont préparées :

| Exemple | 31 | 32 | 33 | 34 | 35 |
|---|---|---|---|---|---|
| tétrachlorhydrate de 2,2-diméthyl-N,N'-bis(2-méthyl-4- aminophényl)propane-1,3-diamine (6) | 10⁻³ mole | 10⁻³ mole | 10⁻³ mole | 10⁻³ mole | 10⁻³ mole |
| 5-Amino-2-méthyl-phénol | 10⁻³ mole | | | | |
| 1H-Indol-6-ol | | 10⁻³ mole | | | |
| 2-Amino-pyridin-3-ol | | | 10⁻³ mole | | |
| chlorhydrate du 2-(2,4-Diamino- phénoxy)-éthanol | | | | 10⁻³ mole | |
| chlorhydrate du 3-Amino-2-chloro-6- méthyl-phénol | | | | | 10⁻³ mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (2) tel que défini précédemment | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90% de blancs. Après 30 minutes de pose, les mèches sont rincées, lavées avec un shampooing standard, rincées à nouveau puis séchées.

Les nuances obtenues figurent dans le tableau ci-dessous :

| Exemple | 31 | 32 | 33 | 34 | 35 |
|---|---|---|---|---|---|
| Nuance observée | violet- rouge | brun rouge | orangé | bleu intense | gris violet |

## Revendications

1. Para-phénylènediamine de formule (I) suivante et ses sels d'addition correspondant : 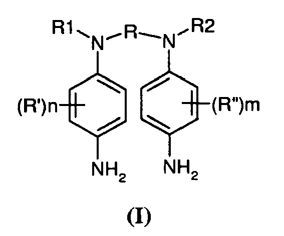 dans laquelle :
• R représente
un radical alkylène en C₃-C₁₅ ramifié,
• R₁ et R₂ représentent, indépendamment l'un de l'autre,
- un atome d'hydrogène,
- un radical alkyle en C₁-C₆ linéaire ou ramifié,
- un radical alkyle en C₁-C₆ linéaire ou ramifié substitué par un ou plusieurs radicaux hydroxy, alkoxy en C₁-C₄, amino, monoalkyl amino en C₁-C₄, dialkylamino en C₁-C₄,
• R' et R" représentent, indépendamment l'un de l'autre,
- un radical alkyle en C₁-C₆,
- un radical alcoxy en C₁-C₆,
- un radical hydroxy-alcoxy en C₁-C₆,
- un radical alcoxy(C₁-C₆)alkyle en C₁-C₆,
- un radical mono ou poly-hydroxy alkyle en C₁-C₆,
• n et m représentent, indépendamment l'un de l'autre, un entier compris entre 0 et 4.

2. Para-phénylènediamine selon la revendication 1 dans laquelle les para-phénylènediamines de formule (I) sont telles que R est un radical alkylène ramifié en C₄-C₁₁.

3. Para-phénylènediamine selon la revendication 1 ou 2 dans laquelle les para-phénylènediamines de formule (I) sont telles R₁ et R₂ représentent indépendamment un atome d'hydrogène ou un groupe alkyle en C₁-C₄ pouvant être substitué.

4. Para-phénylènediamine selon l'une quelconque des revendications 1 à 3 dans laquelle les para-phénylènediamines de formule (I) sont choisies parmi :
| | |
|---|---|
| 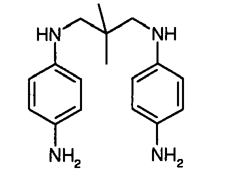 | 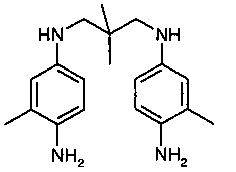 |
| 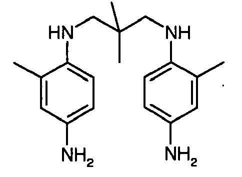 | 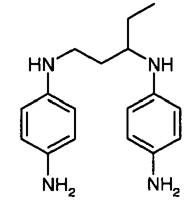 |
| 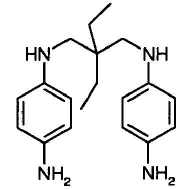 | 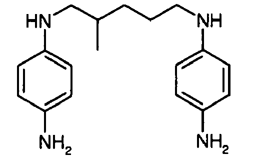 |
| 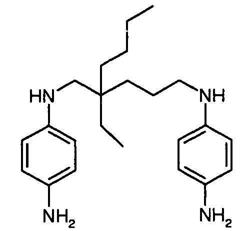 | 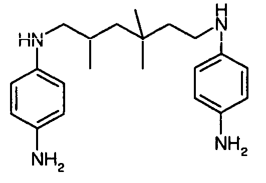 |
| 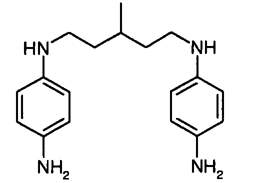 | |

5. Para-phénylènediamine selon l'une quelconque des revendications 1 à 4, **caractérisé en ce que** les sels d'addition avec un acide des para-phénylènediamines de formule générale (I) sont choisis parmi l'acide chlorhydrique, l'acide bromhydrique, l'acide sulfurique, l'acide citrique, l'acide succinique, l'acide tartrique, l'acide lactique, l'acide para-toluènesulfonique, l'acide benzènesulfoniques, l'acide phosphorique et l'acide acétique , ces composés pouvant éventuellement être sous forme de solvates.

6. Composition de coloration comprenant dans un milieu approprié à la teinture au moins une base d'oxydation du type para-phénylènediamine de formule (I) telle que définie à l'une quelconque des revendications 1 à 5.

7. Composition selon la revendication 6 dans laquelle la quantité en para-phénylènediamine de formule (I) est comprise entre 0,0001 % et 20 % en poids par rapport au poids total de la composition, de préférence entre 0,01 % et 10 %.

8. Composition selon la revendication 5, 6 ou 7 comprenant de plus au moins un coupleur d'oxydation.

9. Composition selon la revendication 8 dans laquelle le coupleur d'oxydation est choisi parmi les méta-phénylènediamines, les méta-aminophénols, les méta-diphénols, les coupleurs naphtaléniques et les coupleurs hétérocycliques ainsi que leurs sels d'addition.

10. Composition selon l'une quelconque des revendications 6 à 9 comprenant de plus au moins une base d'oxydation additionnelle différente des bases d'oxydation de formule (I).

11. Composition selon la revendication 10 dans laquelle les bases d'oxydation peuvent être choisies parmi les para-phénylènediamines, les bis-phénylalkylènediamines, les para-aminophénols, les ortho-aminophénols et les bases hétérocycliques et leurs sels d'addition.

12. Composition selon l'une quelconque des revendications 6 à 11 comprenant au moins un colorant direct.

13. Composition selon l'une quelconque des revendications 6 à 12 dans laquelle le milieu approprié pour la teinture est constitué par de l'eau contenant éventuellement au moins un solvant organique.

14. Composition selon la revendication 13 dans laquelle le solvant organique est choisi parmi les alcools inférieurs en C₁-C₄, ramifiés ou non, ainsi que les alcools aromatiques et leurs mélanges.

15. Composition selon l'une quelconque des revendications 6 à 14 comprenant au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

16. Composition selon la revendication 15 **caractérisée en ce que** la quantité en adjuvant cosmétique est comprise pour chacun d'eux entre 0,01 et 20% en poids par rapport au poids total de la composition.

17. Composition selon l'une quelconque des revendications précédentes comprenant un agent oxydant.

18. Procédé de teinture des fibres kératiniques **caractérisée par le fait qu'**on applique sur les fibres au moins une composition selon l'une quelconque des revendications 6 à 16 pendant une durée suffisante pour développer la coloration désirée en présence d'un agent oxydant.

19. Dispositif à plusieurs compartiments dans lequel le premier compartiment contient une composition tinctoriale pour la coloration des fibres kératiniques telle que définie à l'une quelconque des revendications 6 à 18 et un deuxième compartiment contient un agent oxydant.

20. Procédé de préparation des para-phénylènediamines de formule (I) par réduction des composés nitrés de formule (II) : 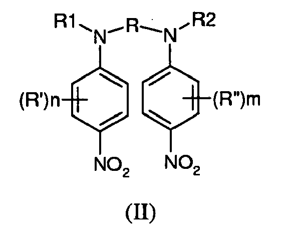 dans laquelle R₁, R₂, R, R', R", n et m sont tels que définis à l'une quelconque des revendications précédentes.

21. Composé nitré de formule (II) 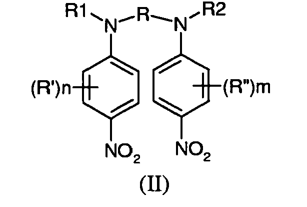 dans laquelle R₁, R₂, X, Y, R, R', R" n, m sont tels que définis à l'une quelconque des revendications précédentes à l'exception du 1,2-propanediamine N, N'-bis(4-nitrophenyl).

## Claims

1. Para-phenylenediamine of the following formula (I) and its corresponding salts of addition: 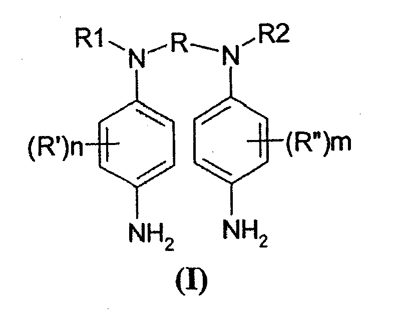 in which:
• R represents a branched C₃-C₁₅ alkylene radical,
• R₁ and R₂ represent, independently of one another,
- a hydrogen atom,
- a linear or branched C₁-C₆ alkyl radical,
- a linear or branched C₁-C₆ alkyl radical substituted by one or more hydroxy, C₁-C₄ alkoxy, amino, C₁-C₄ monoalkylamino, or C₁-C₄ dialkylamino radicals,
• R' and R" represent, independently of one another,
- a C₁-C₆ alkyl radical,
- a C₁-C₆ alkoxy radical,
- a C₁-C₆ hydroxy-alkoxy radical,
- a C₁-C₆ alkoxy(C₁-C₆)alkyl radical,
- a C₁-C₆ mono- or poly-hydroxy alkyl radical,
n and m represent, independently of one another, an integer between 0 and 4.

2. Para-phenylenediamine according to Claim 1, in which the para-phenylenediamines of formula (I) are such that R is a branched C₄-C₁₁ alkylene radical.

3. Para-phenylenediamine according to Claim 1 or 2, in which the para-phenylenediamines of formula (I) are such that R₁ and R₂ represent, independently, a hydrogen atom or a C₁-C₄ alkyl group which may be substituted.

4. Para-phenylenediamine according to any one of Claims 1 to 3, in which the para-phenylenediamines of formula (I) are selected from:
| | |
|---|---|
| 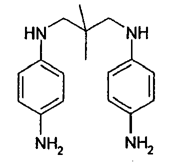 | 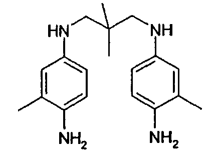 |
| 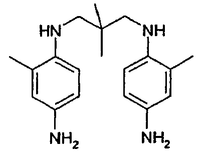 | 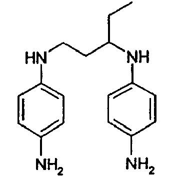 |
| 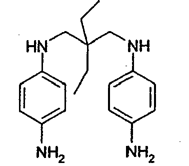 | 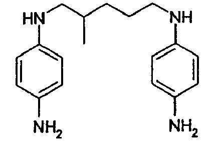 |
| 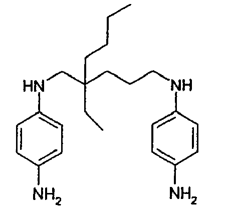 | 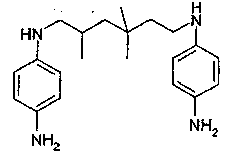 |
| 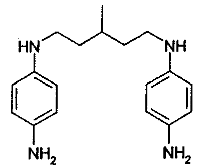 | |

5. Para-phenylenediamine according to any one of Claims 1 to 4, **characterized in that** the salts of addition with an acid, of the para-phenylenediamines of general formula (I) are selected from hydrochloric acid, hydrobromic acid, sulphuric acid, citric acid, succinic acid, tartaric acid, lactic acid, para-toluene-sulphonic acid, benzene-sulphonic acid, phosphoric acid and acetic acid, these compounds optionally being in the form of solvates.

6. Dyeing composition comprising, in a medium that is suitable for dyeing, at least one oxidation base of the type para-phenylenediamine of formula (I) as defined in any one of Claims 1 to 5.

7. Composition according to Claim 6, in which the amount of para-phenylenediamine of formula (I) is between 0.0001 wt.% and 20 wt.% relative to the total weight of the composition, preferably between 0.01% and 10%.

8. Composition according to Claim 5, 6 or 7 additionally containing at least one oxidation coupler.

9. Composition according to Claim 8, in which the oxidation coupler is selected from the metaphenylenediamines, the meta-aminophenols, the meta-diphenols, the naphthalenic couplers and the heterocyclic couplers as well as their salts of addition.

10. Composition according to any one of Claims 6 to 9 additionally containing at least one additional oxidation base different from the oxidation bases of formula (I).

11. Composition according to Claim 10, in which the oxidation bases can be selected from the para-phenylenediamines, the bis-phenylalkylenediamines, the para-aminophenols, the ortho-aminophenols and the heterocyclic bases and their salts of addition.

12. Composition according to any one of Claims 6 to 11 containing at least one direct dye.

13. Composition according to any one of Claims 6 to 12, in which the medium suitable for dyeing comprises water optionally containing at least one organic solvent.

14. Composition according to Claim 13, in which the organic solvent is selected from the linear or branched C₁-C₄ lower alcohols, as well as the aromatic alcohols and mixtures thereof.

15. Composition according to any one of Claims 6 to 14 containing at least one cosmetic additive selected from the antioxidants, penetrants, sequestering agents, perfumes, buffers, dispersants, surfactants, conditioners, film-forming agents, polymers, ceramides, preservatives, lustre agents or opacifiers, vitamins or provitamins.

16. Composition according to Claim 15, **characterized in that** the amount of cosmetic additive is, for each of them, between 0.01 and 20 wt.% relative to the total weight of the composition.

17. Composition according to any one of the preceding claims containing an oxidizing agent.

18. Method of dyeing of keratin fibres, **characterized in that** at least one composition according to any one of Claims 6 to 16 is applied to the fibres for a sufficient time for development of the desired coloration in the presence of an oxidizing agent.

19. Multi-compartment kit in which the first compartment contains a dyeing composition for the dyeing of keratin fibres as defined in any one of Claims 6 to 18 and a second compartment contains an oxidizing agent.

20. Method of production of the para-phenylenediamines of formula (I) by reduction of the nitrogen-containing compounds of formula (II): 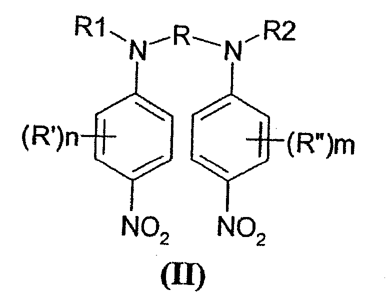 in which R₁, R₂, R, R', R" , n and m are as defined in any one of the preceding claims.

21. Nitrogen-containing compound of formula (II) 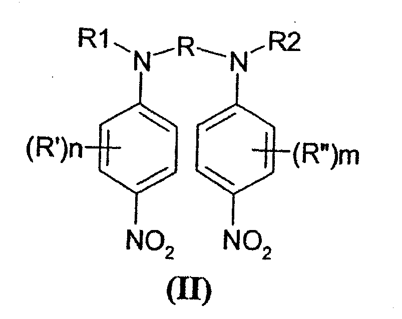 in which R₁, R₂, X, Y, R, R', R", n and m are as defined in any one of the preceding claims with the exception of N,N'-bis(4-nitrophenyl)-1,2-propanediamine.

## Patentansprüche

1. p-Phenylendiamin der folgenden Formel (I) und seine entsprechenden Additionssalze: 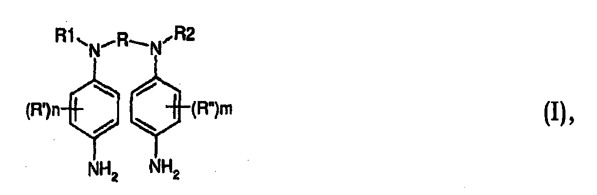 wobei in der Formel:
• R bedeutet
eine verzweigte C₃₋₁-Alkylengruppe,
• R₁und R₂ bedeuten unabhängig voneinander
- ein Wasserstoffatom,
- eine geradkettige oder verzweigte C₁₋₆-Alkylgruppe,
- eine geradkettige oder verzweigte C₁₋₆-Alkylgruppe, die mit einer oder mehreren Gruppen Hydroxy, C₁₋₄-Alkoxy, Amino, C₁₋₄-Monoalkylamino, C₁₋₄-Dialkylamino substituiert ist,
• R' und R" bedeuten unabhängig voneinander
- eine C₁₋₆-Alkylgruppe,
- eine C₁₋₆-Alkoxygruppe,
- eine C₁₋₆-Hydroxyalkoxygruppe,
- eine Alkoxy(C₁₋₆)alkyl(C₁₋₆)gruppe,
- eine Mono- oder Polyhydroxyalkylgruppe mit 1 bis 6 Kohlenstoffatomen,
• n und m bedeuten unabhängig voneinander O oder eine ganze Zahl von 1 bis 4.

2. p-Phenylendiamin nach Anspruch 1, wobei die p-Phenylendiamine der Formel (I) so vorliegen, dass R eine verzweigte C₄₋₁₁-Alkylengruppe bedeutet.

3. p-Phenylendiamin nach Anspruch 1 oder 2, wobei die p-Phenylendiamine der Formel (I) so vorliegen, dass R₁ und R₂ unabhängig voneinander ein Wasserstoffatom oder eine C₁₋₄-Alkylgruppe bedeuten, die substituiert sein kann.

4. p-Phenylendiamin nach einem der Ansprüche 1 bis 3, wobei die p-Phenylendiamine der Formel (I) ausgewählt sind unter:
| | |
|---|---|
| 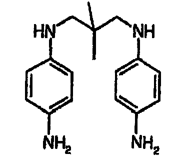 | 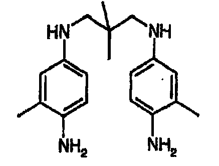 |
| 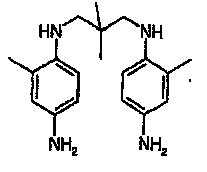 | 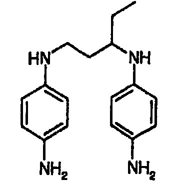 |
| 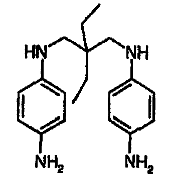 | 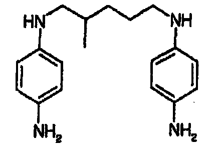 |
| 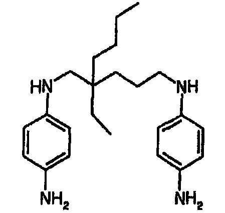 | 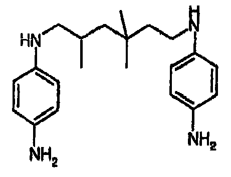 |
| 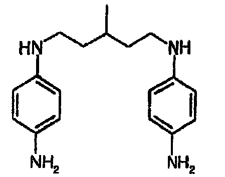 | |

5. p-Phenylendiamin nach einem der Ansprüche 1 bis 4, **dadurch gekennzeichnet, dass** die Additionssalze der p-Phenylendiamine der allgemeinen Formel (I) mit einer Säure ausgewählt sind unter den Salzen mit Salzsäure, Bromwasserstoffsäure, Schwefelsäure, Citronensäure, Bernsteinsäure, Weinsäure, Milchsäure, p-Toluolsulfonsäure, Benzolsulfonsäure, Phosphorsäure und Essigsäure, wobei diese Verbindungen gegebenenfalls in Form der Solvate vorliegen können.

6. Zusammensetzung zum Färben, die in einem zum Färben geeigneten Medium mindestens eine Oxidationsbase vom Typ der p-Phenylendiamine der Formel (I) nach einem der Ansprüche 1 bis 5 enthält.

7. Zusammensetzung nach Anspruch 6, wobei der Mengenanteil des p-Phenylendiamins der Formel (I) im Bereich von 0,0001 bis 20 Gew.-%, bezogen auf das Gesamtgewicht der Zusammensetzung, und vorzugsweise 0,01 bis 10 % liegt.

8. Zusammensetzung nach Anspruch 5, 6 oder 7, die ferner mindestens einen oxidativen Kuppler enthält.

9. Zusammensetzung nach Anspruch 8, wobei der oxidative Kuppler unter den m-Phenylendiaminen, m-Aminophenolen, m-Dihydroxybenzolen, Naphthalinkupplern und heterocyclischen Kupplern sowie deren Additionssalzen ausgewählt ist.

10. Zusammensetzung nach einem der Ansprüche 6 bis 9, die ferner mindestens eine ergänzende, von den Oxidationsbasen der Formel (I) verschiedene Oxidationsbase enthält.

11. Zusammensetzung nach Anspruch 10, wobei die Oxidationsbasen unter den p-Phenylendiaminen, Bis-phenylalkylendiaminen, p-Aminophenolen, o-Aminophenolen und heterocyclischen Basen und deren Additionssalzen ausgewählt sein können.

12. Zusammensetzung nach einem der Ansprüche 6 bis 11, die mindestens einen Direktfarbstoff enthält.

13. Zusammensetzung nach einem der Ansprüche 6 bis 12, wobei das zum Färben geeignete Medium aus Wasser besteht, das gegebenenfalls mindestens ein organisches Lösungsmittel enthält.

14. Zusammensetzung nach Anspruch 13, wobei das organische Lösungsmittel unter den verzweigten oder unverzweigten niederen C₁₋₄-Alkoholen sowie den aromatischen Alkoholen und deren Gemischen ausgewählt ist.

15. Zusammensetzung nach einem der Ansprüche 6 bis 14, die mindestens einen kosmetischen Zusatzstoff enthält, der unter den Antioxidantien, Penetrationsmitteln, Maskierungsmitteln, Parfums, Puffern, Dispergiermitteln, grenzflächenaktiven Stoffen, Konditioniermitteln, Filmbildnern, Polymeren, Ceramiden, Konservierungsmitteln, Perlglanzstoffen oder Trübungsmitteln, Vitaminen oder Provitaminen ausgewählt ist.

16. Zusammensetzung nach Anspruch 15, **dadurch gekennzeichnet, dass** der Mengenanteil des kosmetischen Zusatzstoffes für jeden von ihnen im Bereich von 0,01 bis 20 Gew.-%, bezogen auf das Gesamtgewicht der Zusammensetzung, liegt.

17. Zusammensetzung nach einem der vorhergehenden Ansprüche, die ein Oxidationsmittel enthält.

18. Verfahren zum Färben von Keratinfasern, **dadurch gekennzeichnet, dass** auf die Fasern mindestens eine Zusammensetzung nach einem der Ansprüche 6 bis 16 während einer Zeitspanne aufgebracht wird, die ausreichend ist, um die gewünschte Färbung in Gegenwart eines Oxidationsmittels zu bilden.

19. Vorrichtung mit mehreren Abteilungen, wobei die erste Abteilung eine Farbmittelzusammensetzung zum Färben von Keratinfasern nach einem der Ansprüche 6 bis 18 und eine zweite Abteilung ein Oxidationsmittel enthält.

20. Verfahren zur Herstellung von p-Phenylendiaminen der Formel (I) durch Reduktion von nitrierten Verbindungen der Formel (II): 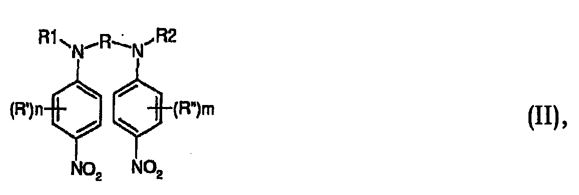 worin R₁, R₂, R, R', R", n und m die in einem der vorhergehenden Ansprüche angegebenen Bedeutungen aufweisen.

21. Nitrierte Verbindung der Formel (II) 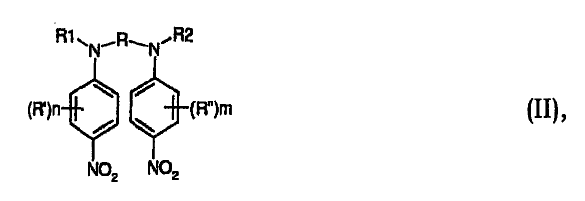 worin R₁, R₂, X, Y, R, R', R", n, m die in einem der vorhergehenden Ansprüche angegebenen Bedeutungen aufweisen, wobei das N,N'-Bis(4-nitrophenyl)-1,2-propandiamin ausgenommen ist.
